# EUROPÄISCHE PATENTSCHRIFT

(11) **EP 1 205 463 B1**
(45) Veröffentlichungstag und Bekanntmachung des Hinweises auf die Patenterteilung: **04.08.2004**
(21) Anmeldenummer: 01124548.7
(22) Anmeldetag: 13.10.2001
(51) Int. Cl.: C07C 45/72

(54) **Verfahren zur Herstellung von Aldolen unter Verwendung eines mikrostrukturierten Reaktionssystems**
Process for the preparation of aldols using a microstructured reaction system
Procédé pour la préparation d' aldols utilisant un système réactionnel microstructuré

(30) Priorität: 07.11.2000 DE 10055758
(43) Veröffentlichungstag der Anmeldung: 15.05.2002
(73) Patentinhaber: Siemens Axiva GmbH & Co. KG, 65926 Frankfurt am Main (DE)
(72) Erfinder: Leipprand, Inga, 65439 Flörsheim (DE); Lahrs, Thorsten, 61118 Bad Vilbel (DE)
(74) Vertreter: Berg, Peter, Dipl.-Ing.

(56) Entgegenhaltungen:
- WO-A-00/51720
- WO-A-95/30476
- US-A- 4 476 324
- US-A- 4 701 562
- US-A- 4 704 478
- NONDEK, L.: "A tubular micro-reactor for measurement of the kinetics of liquid phase heterogeneous reactions under pressure" J. RES. INST. CATAL., HOKKAIDO UNIV. (1979), 27(1), 7-15 , XP001053360
- MOGGI, PIETRO ET AL: "Gas phase aldol condensation of butyraldehyde to 2-ethylhexenal" APPL. CATAL. (1991), 68(1-2), 285-300 , XP001053321

## Beschreibung

Die Erfindung betrifft ein Verfahren zur Herstellung von Aldolen, bei dem man Aldehyde und/oder Ketone katalytisch umsetzt.

Die Herstellung von Aldolen durch Umsetzung von Aldehyden mit sich selbst oder mit anderen Aldehyden, Umsetzung von Aldehyden mit Ketonen oder von Ketonen mit sich selbst oder mit anderen Ketonen unter Verwendung eines basischen Katalysators gehört zu den Standardreaktionen der organischen Synthese. Diese Reaktionen werden daher auch allgemein als Aldolreaktion bezeichnet (vgl. ORGANIKUM, 18. AUFLAGE, DVW, BERLIN 1990). Auch in der Produktion der chemischen Industrie wird die Aldolreaktion eingesetzt, z. B. zur Herstellung von Acetaldol aus Acetaldehyd (vgl. ULLMANN'S ENCYCLOPEDIA OF INDUSTRIAL CHEMISTRY, SIXTH EDITION, 1999 ELECTRONIC RELEASE). Im Anschluss an die Aldolreaktion kann eine Dehydratisierung des Aldols zu einem ungesättigten Aldehyd oder Keton durchgeführt werden.

Aus der US 4,701,562 und der US 4,704,478 ist es jeweils bekannt, die Herstellung von Aldolen in einem so genannten Mikroreaktor durchzuführen, der aus einem Rohr mit einem Durchmesser von 9 mm besteht und eine Katalysatorfüllung aus nichtzeolithischen Molsieben enthält.

Die US 4,476,324 beschreibt die Verwendung von Tonmineralien in einem Reaktor von 1 inch zur Aldolherstellung.

Aus der WO 95/30476 ist es bekannt, chemische Reaktionen in einem durch Mikrotechnik hergestellten mikrostrukturierten Reaktionssystem durchzuführen.

Die WO 00/51720 offenbart eine Anordnung zur Untersuchung von verschiedenen Katalysatoren, wobei mindestens vier Mikroreaktoren vorhanden sind, in welche mindestens vier Katalysatormaterialien eingebracht werden. Die untersuchten Katalysatoren können zur Beschleunigung einer Vielzahl unterschiedlicher chemischer Reaktionen, darunter auch die Aldol-Kondensation, dienen.

L. Nondek beschreibt in J. Res. Inst. Catalysis, Hokkaido Univ., 27(1) (1979), Seiten 7 - 16 die Aldolherstellung in einem 4 mm weiten Stahlrohr, das mit feinen Partikeln eines Katalysators gefüllt ist.

P. Moggi et al. beschreiben in Applied Catalysis, 68 (1991), Seiten 285 - 300 die Aldolherstellung in einem so genannten Mikroreaktor mit einem inneren Durchmesser von 10 mm und einer Katalysatorfüllung.

Die Verfahren nach dem Stand der Technik weisen verschiedene Beschränkungen bei der Durchführung der Reaktion auf. Zum einen kann die maximale Reaktionstemperatur durch die niedrigste Siedetemperatur einer der Komponenten begrenzt sein, wenn eine drucklose Betriebsweise angesetzt wird. Dadurch wird die Reaktionsgeschwindigkeit und somit auch die Raum-Zeit-Ausbeute beschränkt; längere Verweilzeiten können die Selektivität herabsetzen. Weiterhin treten Beschränkungen auf, welche durch die Wärmeabfuhrleistung der eingesetzten Apparate und durch sicherheitstechnische Aspekte aufgrund der exothermen Reaktion gegeben sind. Dazu zählen z. B. verlangsamte Dosierung oder Einsatz einer geringen Katalysatormenge. Auch in diesen Fällen wird die Umsetzungsgeschwindigkeit und somit auch die Raum-Zeit-Ausbeute beschränkt und die Selektivität beeinträchtigt

Der Erfindung liegt die Aufgabe zugrunde ein Verfahren zur Aldolherstellung mit erhöhter Raum-Zeit-Ausbeute und verbessertem Umsatz oder Selektivität bereitzustellen.

Diese Aufgabe wird erfindungsgemäß dadurch gelöst, dass man bei dem Verfahren der eingangs genannten Art die Umsetzung in Kanälen eines durch Mikrotechnik hergestellten mikrostrukturierten Reaktionssystems durchführt, wobei die Kanäle einen Strömungsquerschnitt zwischen 5000 µm² und 5 mm² aufweisen und auf ihren Wänden ein heterogener Katalysator durch Bedampfen oder Abscheiden aufgebracht ist.

Besondere Ausführungsformen des erfindungsgemäßen Verfahrens sind in den abhängigen Ansprüchen offenbart.

Als mikrostrukturierte Reaktionssysteme im Sinne der Erfindung sind Mikrobauteile definiert, die mittels Mikrotechnik hergestellt wurden. Die charakteristischen Abmessungen der inneren Strukturen der Mikrobauteile, wie Kanäle, liegen typischerweise im sub-mikrometer bis sub-millimeter Bereich (vgl. ULLMANN'S ENCYCLOPEDIA OF INDUSTRIAL CHEMISTRY, SIXTH EDITION, 1999 ELECTRONIC RELEASE). Dabei werden die mikrostrukturierten Bauteile zur schnellen Durchmischung der Edukte und/oder zur exakten Einstellung der gewünschten Temperatur eingesetzt. Die Temperaturführung betrifft das Aufheizen der Edukte, das Einhalten einer konstanten Reaktionstemperatur und das Abkühlen der Reaktionslösung zum Beenden der Reaktion.

Als Mikrobauteile eignen sich insbesondere Mikromischer gemäß DE 197 46 583 A1 sowie US 5,904,424 oder Mikrowärmeaustauscher, die auch als Reaktoren verwendbar sind (z. B. gemäß DE 195 41 266 A1). Diese Bauteile eignen sich auch zum schnellen Aufheizen der Rohstoffmischung.

Weiterhin zeichnet sich das erfindungsgemäße Verfahren dadurch aus, dass ein heterogener Katalysator durch Bedampfen der Wände der Kanäle oder durch chemisches oder physikalisches Abscheiden, insbesondere durch elektrochemisches Ab-scheiden, in das mikrostrukturierte System eingebracht ist. Dabei werden vorzugsweise Katalysatoren eingesetzt, die in Literatur und Patenten beschrieben sind, insbesondere basische Anionentauscher, Metalloxide von Mo, W, Ca, MG und Al, basische Zeolithe (Process and catalysts for the preparation of polyhydric alcohols by the aldol condensation of aldehydes followed by hydrogenation, E. Paatero, E. Nummi; L. Lindfors; H. Nousiainen; J. Hietala; L. Lahtinen; R. Haakana; (Neste Oy, Finland), WO 97-FI835, 19971230; Method for producing alkenal by crossed aldol condensation of saturated aldehydes using zeolite-supported base catalyst; M. Ichikawa; R. Oonishi; M. Fukui; H. Harada (Chisso Corp, Japan), JP 95-218842, 19950828; Catalyst for carrying out the aldol condensation; Reichle, Walter Thomas (Union Carbide Corp., USA) US-76-657568, 19760212).

Die Vorteile des erfindungsgemäßen Verfahrens liegen darin, dass das verwendete Reaktionssystem eine Vielzahl mikrostrukturierter Kanäle enthält, die es ermöglichen, dass die Reaktion unter verbesserten Reaktionsbedingungen durchgeführt werden kann. Die charakteristischen Abmessungen der Mikrokanäle, das heißt der Strömungsquerschnitt, betragen dabei 5000 µm² bis 5 mm². Diese erfindungsgemäße Verbesserung der Reaktionsbedingungen führt dazu, dass die Raum-Zeit-Ausbeute, der Umsatz oder die Selektivität der Reaktion erhöht werden kann.

Die Reaktion wird vorzugsweise in einer kontinuierlichen Betriebsweise in der Flüssigphase durchgeführt. Dabei wird ein Aldehyd R₁CHO(R₁=C1-12-alkyl, C3-C12-cycloalkyl, aryl, C≤14 aralkyl) mit sich selbst oder einem zweiten Aldehyd R₂CHO (R₂=H, C1-12-alkyl, C3-C12-cycloalkyl, aryl, C≤14 aralkyl) oder mit einem Keton R₁R₂CO (R₁, R₂=C1-12-alkyl, C3-C12-cycloalkyl, aryl, C≤14 aralkyl) oder es wird ein Keton R₁R₂CO (R₁, R₂=C1-12-alkyl, C5-C12-cycloalkyl, aryl, C≤14 aralkyl) mit sich selbst oder einem anderen Keton R₃R₄CO (R₃, R₄=C1-12-alkyl, C3-C12-cycloalkyl, aryl, C≤14 aralkyl) umgesetzt. Sämtliche genannten Aldehyde und Ketone können jeweils unabhängig voneinander gleich oder verschieden substituiert oder nicht substituiert sein.

Zur Erhaltung der flüssigen Phase bei höheren Temperaturen kann die Reaktion unter Druck zwischen 1 und 50 bar absolut durchgeführt werden. Die Mikrostrukturierung erlaubt eine sehr exakte Einstellung der gewünschten Temperatur, wobei auch große Wärmemengen, die bei exothermen Reaktionen freigesetzt werden, sicher abgeführt werden können. Die Reaktion kann bei Temperaturen aus dem Bereich von -10 bis 250°C, bevorzugt von 50 bis 200°C durchgeführt werden. Die Verweilzeit der Aldehyde oder Ketone oder Aldole in den Kanälen des mikrostrukturierten Systems beträgt dabei 0,001 bis 1000 s, bevorzugt 0,01 bis 100 s. Zur Erweiterung der Reaktionszeit kann das mikrostrukturierte System durch eine Verweilzeitstrecke, die nicht mikrostrukturiert ist, ergänzt werden.

## Patentansprüche

1. Verfahren zur Herstellung von Aldolen, bei dem man Aldehyde und/oder Ketone katalytisch umsetzt, **dadurch gekennzeichnet, dass** man die Umsetzung in Kanälen eines durch Mikrotechnik hergestellten mikrostrukturierten Reaktionssystems durchführt, wobei die Kanäle einen Strömungsquerschnitt zwischen 5000 µm² und 5 mm² aufweisen und auf ihren Wänden ein heterogener Katalysator durch Bedampfen oder Abscheiden aufgebracht ist.

2. Verfahren nach einem oder mehreren der vorstehenden Ansprüchen, wobei der Katalysator ausgewählt ist aus der Gruppe bestehend aus basischen Anionentauschern, Metalloxiden von Mo, W, Ca, Mg und Al, sowie basischen Zeolithen.

3. Verfahren nach einem oder mehreren der vorstehenden Ansprüchen, wobei man die Umsetzung bei Drücken zwischen 1 und 50 bar absolut und zwischen -10 und 250°C durchführt.

4. Verfahren nach einem oder mehreren der vorstehenden Ansprüchen, wobei man ein Aldehyd R₁CHO mit sich selbst oder einem zweiten Aldehyd R₂CHO oder mit einem Keton R₁R₂CO umsetzt oder ein Keton R₁R₂CO mit sich selbst oder einem anderen Keton R₃R₄CO umsetzt, wobei R₁ bis R₄, jeweils gleich oder verschieden, unabhängig voneinander folgende Bedeutung haben können:
R₁: C1-12-alkyl, C3-C12-cycloalkyl, aryl, C≤14 aralkyl, jeweils substituiert oder nichtsubstituiert;
R₂: C1-12 -alkyl, C3-C12-cycloalkyl, aryl, C≤14 aralkyl, jeweils substituiert oder nichtsubstituiert, H;
R₃: C1-12-alkyl, C3-C12-cycloalkyl, aryl, C≤14 aralkyl, jeweils substituiert oder nichtsubstituiert;
R₄: C1-C12-alkyl, C3-C12-cycloalkyl, aryl, C≤14 aralkyl, jeweils substituiert oder nichtsubstituiert.

5. Verfahren nach einem oder mehreren der vorstehenden Ansprüchen, wobei die Verweilzeit der Aldehyde oder Ketone oder Aldole in den Kanälen 0,001-1000 s beträgt.

6. Verfahren nach einem oder mehreren der vorstehenden Ansprüchen, wobei das Reaktionssystem durch eine Verweilzeitstrecke, die nicht mikrostrukturiert ist, erweitert ist.

## Claims

1. Process for preparing aldols by catalytic reaction of aldehydes and/or ketones, **characterized in that** it comprises conducting said reaction in channels of a microstructured reaction system manufactured using microtechnology, wherein said channels have a flow cross section of between 5000 µm2 and 5 mm2 and a heterogeneous catalyst is applied to the walls of said channels by vapour deposition or chemical or physical deposition.

2. Process of one or more of the preceding claims, wherein said catalyst is selected from the group consisting of basic anion exchangers, metal oxides of Mo, W, Ca, Mg and Al and basic zeolites.

3. Process of one or more of the preceding claims, wherein said reaction is conducted at pressures between 1 and 50 bar absolute and between -10 and 250°C.

4. Process of one or more of the preceding claims, wherein an aldehyde R1CHO is reacted with itself or with a second aldehyde R2CHO or with a ketone R1R2CO or a ketone R1R2CO is reacted with itself or another ketone R3R4CO and R1 to R4, which are each identical or different, independently can have the following meanings:
R₁: C1-12-alkyl, C3-C12-cycloalkyl, aryl, C<14 aralkyl, each substituted or unsubstituted;
R₂: C1-12-alkyl, C3-C12-cycloalkyl, aryl, C<14 aralkyl, each substituted or unsubstituted, H;
R₃: C1-12-alkyl, C3-C12-cycloalkyl, aryl, C<14 aralkyl, each substituted or unsubstituted;
R₄: C1-12-alkyl, C3-C12-cycloalkyl, aryl, C<14 aralkyl, each substituted or unsubstituted.

5. Process of one or more of the preceding claims, wherein the residence time of said aldehydes or ketones or aldols in said channels is in the range from 0.001 to 1000 s.

6. Process of one or more of the preceding claims, wherein said reaction system is supplemented by a delay time zone which is not microstructured.

## Revendications

1. Procédé de préparation d'aldols, dans lequel on fait réagir catalytiquement des aldéhydes et/ou des cétones, **caractérisé en ce que** l'on effectue la réaction dans des canaux d'un système de réaction structuré microscopiquement fabriqué par une technique microscopique, les canaux ayant une section transversale d'écoulement comprise entre 5.000 µm² et 5 mm² et un catalyseur hétérogène étant déposé sur leurs parois par évaporation ou par dépôt.

2. Procédé suivant l'une ou plusieurs des revendications précédentes, dans lequel le catalyseur est choisi dans le groupe constitué d'échangeurs d'anions basiques, d'oxydes métalliques de Mo, W, Ca, Mg et Al ainsi que de zéolites basiques.

3. Procédé suivant l'une ou plusieurs des revendications précédentes, dans lequel on effectue la réaction sous des pressions comprises en 1 et 50 bar absolus et entre -10 et 250°C.

4. Procédé suivant l'une ou plusieurs des revendications précédentes, dans lequel on fait réagir un aldéhyde R₁CHO sur soi-même ou sur un deuxième aldéhyde R₂CHO ou sur une cétone R₁R₂CO ou on fait réagir une cétone R₁R₂CO sur soi-même ou sur une autre cétone R₃R₄CO, R₁ à R₄, qui sont respectivement identiques ou différents, pouvant avoir indépendamment les uns des autres, la signification suivante :
R₁ : alcoyle ayant de 1 à 12 atomes de carbone, cycloalcoyle ayant de 3 à 12 atomes de carbone, aryle, aralcoyle ayant un nombre d'atomes de carbone 14. respectivement substitués ou non substitués ;
R₂ : alcoyle ayant de 1 à 12 atomes. de carbone, cycloalcoyle ayant de 3 à 12 atomes de carbone, aryle, aralcoyle ayant un nombre d'atomes de carbone 14, respectivement substitués ou non substitués, H ;
R₃ : alcoyle ayant de 1 à 12 atomes de carbone, cycloalcoyle ayant de 3 à 12 atomes de carbone, aryle, aralcoyle ayant un nombre d'atomes de carbone 14, respectivement substitués ou non substitués :
R₄ : alcoyle ayant de 1 à 12 atomes de carbone, cycloalcoyle ayant de 3 à 12 atomes de carbone, aryle, aralcoyle ayant un nombre d'atomes de carbone 14, respectivement substitués ou non substitués.

5. Procédé suivant l'une ou plusieurs des revendications précédentes, dans lequel la durée de séjour des aldéhydes ou des cétones ou des aldols dans les canaux est comprise entre 0,001 et 1000 s.

6. Procédé suivant l'une ou plusieurs des revendications précédentes, dans lequel on agrandit le système de réaction par une section de temps de séjour, qui n'est pas structurée microcopiquement.
